# EUROPEAN PATENT APPLICATION

(11) **EP 4 253 980 A1**
(43) Date of publication of application: **04.10.2023**
(21) Application number: 22164911.4
(22) Date of filing: 29.03.2022
(51) Int. Cl.: G01R 33/36, G01R 33/34

(54) **TUNABLE RADIO FREQUENCY COIL ASSEMBLY AND MAGNETIC RESONANCE SYSTEM COMPRISING THE RF COIL ASSEMBLY**

(71) Applicant: Koninklijke Philips N.V., 5656 AG Eindhoven (NL)
(72) Inventor: LEUSSLER, Christoph Günther, Eindhoven (NL); VERNICKEL, Peter, Eindhoven (NL); FINDEKLEE, Christian, Eindhoven (NL)
(74) Representative: Philips Intellectual Property & Standards

(57) **Abstract**

The invention refers to a Radio Frequency (RF) coil assembly (100) for magnetic resonance imaging. The RF coil assembly (100) contains an RF coil (130) configured to receive and/or transmit an RF signal (122) input and/or output, a detune arrangement (110) configured to receive a control signal (121) and to tune/detune the RF coil (130) to a resonance frequency based on the control signal (121), and a conductor (120) configured to conduct the RF signal (122) and the control signal (121). The detune arrangement (110) is electrically coupled to the RF coil (130) and the conductor (120) is electrically coupled to both the RF coil (130) and to the detune arrangement (110). The tuning/detuning control signal (121) input is transmitted via the same conductor (120) as the RF signal (122) input/output, thereby reducing the number of separate conductors for tuning or detuning. In embodiments of the invention, the detune arrangement (110) comprises at least one Field Effect Transistor (320) and a bias network (310).

## Description

### FIELD OF THE INVENTION

The invention relates to tuning of a Radio Frequency (RF) coil.

Magnetic resonance imaging (MRI) methods utilize the interaction between magnetic fields and nuclear spins in order to form two-dimensional or three-dimensional images are widely used nowadays, notably in the field of medical diagnostics, because for the imaging of soft tissue they are superior to other imaging methods in many respects, do not require ionizing radiation and are usually not invasive.

According to the MRI method in general, the body of the patient to be examined is arranged in a strong, uniform magnetic field B0 whose direction at the same time defines an axis (normally the z-axis) of the co-ordinate system to which the measurement is related. The magnetic field B0 causes different energy levels for the individual nuclear spins in dependence on the magnetic field strength which can be excited (spin resonance) by application of an electromagnetic alternating field (RF field) of defined frequency (so-called Larmor frequency, or MR frequency). From a macroscopic point of view the distribution of the individual nuclear spins produces an overall magnetization which can be deflected out of the state of equilibrium by application of an electromagnetic pulse of appropriate frequency (RF pulse) while the corresponding magnetic field B1 of this RF pulse extends perpendicular to the z-axis, so that the magnetization performs a precession motion about the z-axis. The precession motion describes a surface of a cone whose angle of aperture is referred to as flip angle. The magnitude of the flip angle is dependent on the strength and the duration of the applied electromagnetic pulse. In the example of a so-called 90° pulse, the magnetization is deflected from the z axis to the transverse plane (flip angle 90°).

After termination of the RF pulse, the magnetization relaxes back to the original state of equilibrium, in which the magnetization in the z direction is built up again with a first time constant T1 (spin lattice or longitudinal relaxation time), and the magnetization in the direction perpendicular to the z-direction relaxes with a second and shorter time constant T2 (spin-spin or transverse relaxation time). The transverse magnetization and its variation can be detected by means of receiving RF antennae (RF coil arrays) which are arranged and oriented within an examination volume of the magnetic resonance examination system in such a manner that the variation of the magnetization is measured in the direction perpendicular to the z-axis. The decay of the transverse magnetization is accompanied by dephasing taking place after RF excitation caused by local magnetic field inhomogeneities facilitating a transition from an ordered state with the same signal phase to a state in which all phase angles are uniformly distributed. The dephasing can be compensated by means of a refocusing RF pulse (for example a 180° pulse). This produces an echo signal (spin echo) in the receiving coils.

In order to realize spatial resolution in the subject being imaged, such as a patient to be examined, constant magnetic field gradients extending along the three main axes are superposed on the uniform magnetic field B0, leading to a linear spatial dependency of the spin resonance frequency. The signal picked up in the receiving antennae (RF coil arrays) then contains components of different frequencies which can be associated with different locations in the body. The signal data obtained via the receiving coils correspond to the spatial frequency domain of the wave-vectors of the magnetic resonance signal and are called k-space data. The k-space data usually include multiple lines acquired of different phase encoding. Each line is digitized by collecting a number of samples. A set of k-space data is converted to an MR image by means of Fourier transformation.

### BACKGROUND OF THE INVENTION

To prevent RF coils from being damaged by powerful RF pulses and to increase signal to noise ratio, RF coils are electronically tuned or detuned.

The German utility model DE202018002146U describes a magnetic resonance antenna with antenna resonant circuit elements and with at least one high frequency switching element. The high frequency switching element for determining radio frequency of the antenna resonant circuit elements, is switched between a transmissive state and an impermeable state to change the natural resonance frequency of the magnetic resonance antenna. The high frequency switching element is selected from a field effect transistor and/or one micro-electromechanical system.

### SUMMARY OF THE INVENTION

It is an object of the invention to provide an RF coil assembly with an improved architecture for tuning or detuning the RF coil.

The invention is defined by the independent claims. Advantageous embodiments are defined in the dependent claims.

The improved RF assembly architecture for tuning or detuning the RF coil reduces the number of conductors to/from the assembly. Performance of the RF assembly may be enhanced by the invention, e.g. due to reduced interference between conductor lines. In addition, the cost and bulkiness of the RF assembly may advantageously be decreased.

In a first aspect of the invention an RF coil assembly for magnetic resonance imaging is presented. The RF coil assembly contains an RF coil configured to receive and/or transmit an RF signal input and/or output, a detune arrangement configured to receive a control signal and to tune/detune the RF coil to a resonance frequency based on the control signal, and a conductor configured to conduct the RF signal and the control signal. The detune arrangement is electrically coupled to the RF coil and the conductor is electrically coupled to both the RF coil and to the detune arrangement. The tuning/detuning control signal input is transmitted via the same conductor as the RF signal input/output.

This means that the tuning or detuning control signal enters the RF coil assembly on the same conductor that conducts the RF power for transmission or MR signal for reception, thereby reducing the number of connections between RF coil and MR system. By reducing or eliminating additional feeding lines it may be possible to reduce noise, cost and/or bulkiness of the assembly.

In embodiments of the invention, the detune arrangement comprises at least one Field Effect Transistor (FET) and a bias network. The bias network is configured to switch the FET between an open and a closed state in response to the control signal. The detune arrangement may thereby regulate tuning or detuning of the RF coil. FETs are advantageous in combination with the common conductor since they require very low power to switch and no or very low continuous current is needed to maintain the state of the FET. The FETs may be e.g. Gallium Nitride FETs, Gallium Nitride high-electron-mobility transistors, enhancement mode FETs, FETs in a cascode circuit etc.

The current may typically be on the order of 10-100 µA or smaller such as 0.1-10 µA. Depending on the FET it may be higher or lower. With a FET gate voltage in the range of e.g. 0.1-10 V the required power is very low. Even with potentially higher gate voltages the required power is low. It is expected that future developments of FETs may further improve performance during switching and in the on and off states. E.g. via improved enhancement mode and/or cascode FETs in a single housing.

In embodiments of the invention, the common conductor is galvanically isolated from the FET gate in the detune arrangement. The RF coil assembly is configured to divide a voltage of the control signal over resonance capacitors on the RF coil and a FET gate capacitance in the detune arrangement. By interrupting the galvanic connection, no DC current flows at the detune arrangement and coupling with RF signals may be avoided or reduced. This may lead to improved homogeneity and symmetry of the resonator.

In embodiments of the invention, the detune arrangement is configured such that multiple FETs are switched in parallel in response to the control signal. The nature of FETs or similar switches makes it possible to use multiple switches that are switched in parallel. With such a configuration, an RF coil assembly with multiple switches may be efficiently tuned/detuned with the same control signal input. A single wire connection with the RF coil assembly may be used to control all tuning/detuning switches in parallel.

In embodiments of the invention, the control signal transmitted over the common conductor is a time-shifted pulsed DC signal. The pulsed DC control signal may advantageously be combined with the RF signal as a DC offset at the RF amplifier for transmission through the common conductor and the signals may be separated at the RF coil assembly. With a time-shifted signal the FET switch may be switched in advance such that detuning is active before RF transmission or reception takes place in the RF coil.

In embodiments of the invention, the RF coil assembly is an RF body coil assembly, a transverse electromagnetic resonator assembly, or an RF birdcage coil assembly. The conductor is configured to transmit RF power. Removal of extra feeding lines may be particularly advantageous in this type of transmit coils. Such coils typically have multiple channels, such as e.g. 16 or 32 channels, which require tuning/detuning. By removing extra feeding lines, common mode coupling between coil RF conductors and DC control lines may be avoided or reduced. Such coupling can cause unbalancing of the birdcage resonator and potentially MRI system signal integrity issues.

In embodiments of the invention, the detune arrangement is embedded in a printed circuit board of the RF coil assembly. It is advantageous to avoid separate wires on the RF coil besides the printed circuit boards used for RF conduction.

In embodiments of the invention, the RF coil assembly comprises a safety monitoring arrangement that is configured to monitor the tuning status of the RF coil assembly. Monitoring the tuning status of the RF coil may increase patient safety and measurement integrity. Timely monitoring may also give the possibility to control tuning/detuning, compensate for and/or predict inhomogeneities in RF currents etc.

In embodiments of the invention, the safety monitoring element comprises a sensor and is configured to transmit a monitoring signal from the sensor via the conductor. The monitoring element thereby requires no additional conductors that could otherwise be potential sources of coupling noise on the RF coil assembly. This may also allow real-time feedback signals from sensors.

In other embodiments of the invention, the safety monitoring element comprises a sensor and is configured to transmit a monitoring signal from the sensor wirelessly or via an optical fiber. Such a configuration may provide a mode of communication to monitor the status of the RF coil assembly, without having to add any galvanic conductors from the RF coil assembly for the monitoring signal.

In embodiments of the invention, the detune arrangement is configured to receive a first control signal via the common conductor and a second control signal wirelessly or via an optical fiber. It may be advantageous to have multiple ways of communicating with the detune arrangement, e.g. for safety reasons, without adding any extra galvanic feeding lines to the RF coil assembly.

According to another aspect of the invention, there is provided a Magnetic Resonance system comprising the RF coil assembly.

These and other aspects of the invention will be apparent from and elucidated with reference to the embodiments described hereinafter.

### BRIEF DESCRIPTION OF THE DRAWINGS

Fig. 1 shows a schematic illustration of an RF coil assembly with a common conductor according to embodiments of the invention.
Fig. 2 shows a schematic illustration of voltage division over capacitances on the RF coil assembly according to embodiments of the invention.
Fig. 3 shows a schematic illustration of an RF body transmit coil according to embodiments of the invention.
Fig. 4 shows a schematic illustration of an RF coil assembly electronic circuit according to embodiments of the invention.
Fig. 5 shows a schematic illustration of an RF coil assembly electronic circuit according to embodiments of the invention.
Fig. 6 shows a schematic illustration of an RF coil assembly electronic circuit according to embodiments of the invention.
Fig. 7 shows a schematic illustration of an RF coil assembly with a safety monitoring arrangement according to embodiments of the invention.
Fig. 8 shows a schematic illustration of another RF coil assembly with a safety monitoring arrangement according to embodiments of the invention.

### DESCRIPTION OF EMBODIMENTS

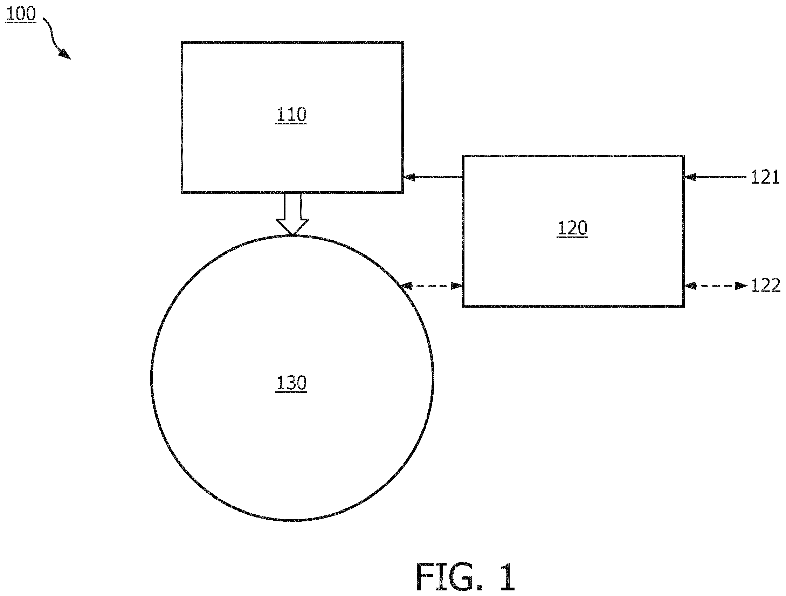

Fig. 1 shows a schematic illustration of an embodiment of the invention. The RF coil assembly 100 includes a transmit and/or receive RF coil 130, a detune arrangement 110 and a common conductor 120. The RF coil 130 receives and/or transmits an RF signal 122 input and/or output for magnetic resonance imaging. The detune arrangement 110 receives a control signal 121 to tune/detune the RF coil 130 to a resonance frequency based on the control signal 121. Both the tuning/detuning control signal 121 input and the RF signal 122 input/output is conducted via the same conductor 120.

In a preferred embodiment, the detune arrangement 110 includes integrated FETs 320 (not shown in Fig.1) and the control signal 121 is a DC signal, such as a time-shifted pulsed DC signal. A time-shifted signal in such an example means that the DC signal has a time shift with respect to the RF transmit or receive signal 122. In one example the FET 320 switch may thereby be switched in advance such that detuning is active before RF transmission or reception takes place in the RF coil 130. The control signal 121 time-shift may be in the order of 0.1 µs but may also be longer or shorter.

Due to the nature of FETs 320 or similar switches it is possible to use multiple switches that are switched in parallel. In such an embodiment, a single wire connection with the RF coil assembly may be used to control all tuning/detuning switches in parallel.

As an example, the RF coil 130 is an RF transmit coil that receives RF power from an RF amplifier in order to create a B1 magnetic field. In this example, the DC control signal 121 enters the RF coil assembly 100 on the same cable as the RF power for transmission, thereby reducing the number of connections between RF coil assembly 100 and MRI system.

The RF amplifier may be extended with a module providing a DC offset for controlling detuning of the RF coil 130 in addition to the RF power. Standard electronic components such as nonmagnetic bias tees, chokes and blocking capacitors may be used to combine the control signal 121 and the RF signal 122 as well as to separate the RF power and pulsed DC control signals 121 inside the RF coil assembly 100.

When no continuous current is required to maintain a state of tuning/detuning, which may be the case when using FETs 320 or similar switches for switching in the detune arrangement 110, there is no need for a direct galvanic connection between the control signal source and the FET gate. The supply line may be interrupted e.g. with the resonance capacitors 210 of the RF coil 130. With the resonance capacitors 210 having a capacitance that is significantly higher than the FET gate capacitance 220, most of the voltage drops over the FET gate so that it can be switched.

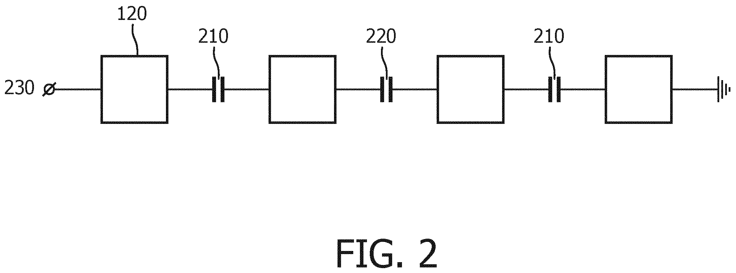

A schematic illustration of how the supply voltage via the conductor 120 is divided is shown in Fig. 2. The resonance capacitors 210 may be in the order of several 10pF whereas the FET gate capacitance 220 may be in the order of 10pF. In the resulting voltage divider still the majority of the voltage will drop over the FET gate such that it can be switched. Many ranges of capacitances are possible. The FET gate capacitance 220 may be between 1-20pF and the resonance capacitors 210 between 50-100pF. However, many different ranges of capacitances of the FET gate and the resonance capacitors 210 are possible, as long as there is sufficient voltage drop over the FET gate to switch the FET state.

In other embodiments, it is conceivable that instead of FETs 320, another type of switch used, such as a material phase change switch or a micro-electromechanical switch.

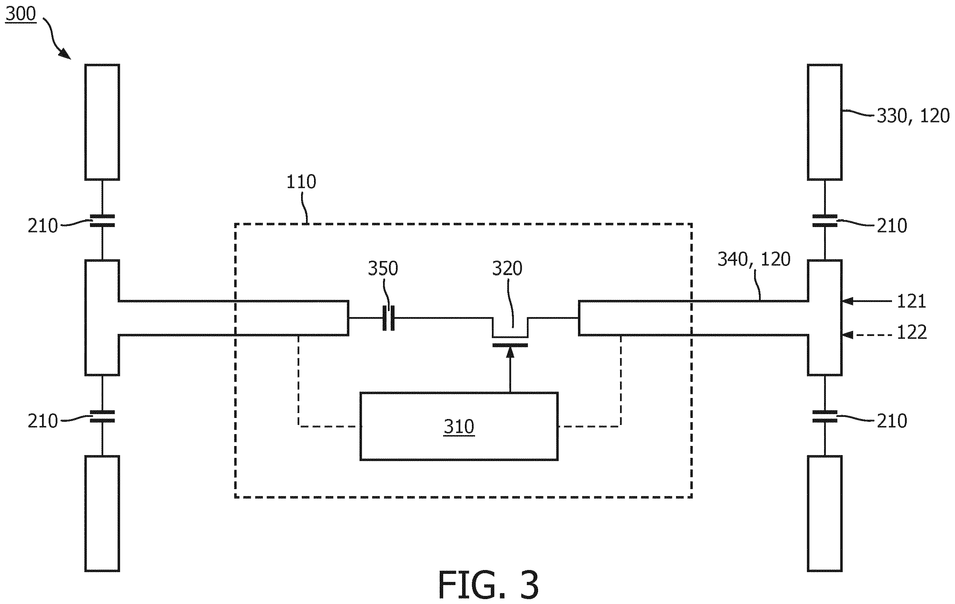

Fig. 3 shows a schematic illustration of an RF transmit body coil 300, according to embodiments of the invention. Removal of extra feeding lines may be particularly advantageous in this type of transmit coils. Such coils may have multiple channels, such as e.g. 16 or 32 channels, which require tuning/detuning. The control signal 121 for the detune arrangement 110 with integrated FET 320 switches is supplied via the RF power feeding cable and printed circuit board conductors 120 of the RF coil 130. No extra DC wire is required. Thus DC cable routing can be avoided for all RF body coils 300 in the field of view. This may help to remove a source of B0 inhomogeneity and mechanical break due to magnetic forces. During transmission the FETs 320, such as depletion mode FETs, may have low resistance to provide detuning without the need for continuous power supply.

As depicted in Fig.3, the control signal 121 to switch the FET 320 with the Bias Network 310 is routed via the ring 330 and rung 340 of the body coil printed circuit board RF conductors 120. In this example, no extra DC supply cable for the detuning arrangement 110 is required. It may also be envisioned that no extra return path for the DC supply of the FET detuning switch is required, as the current is negligible. This current may be on the order of µA.

Since no continuous current is required to maintain a state of tuning/detuning with the FETs 320 for switching in the detune arrangement 110, there is no need for a direct galvanic connection between the control signal source and the FETs 320. The supply line may be interrupted e.g. with the resonance capacitors 210 or detune capacitance 350 of the transmit body coil 300.

The switching voltage control signal 121 can be supplied from the RF amplifier to the RF transmit body coil 300 via the RF cable feeding, such as via a bias Tee and a high-power coax cable. Routing and bridging of the signals on the RF transmit body coil 300 may be realized via components such as RF chokes, bias Tees, capacitors and high ohmic resistors.

In embodiments, an RF screen (not shown in Fig. 3) is also part of the transmit body coil 300. Due to a close proximity, high RF currents are present as mirror or current return currents. The RF screen is split by discrete or distributed capacitors. Such a structure may also allow to generate tuning/detuning control signal input 121 and RF signal 122 via the same conductor 120. For a detune arrangement 110 located in the center of the body coil 300, a short flexible connection supplies the detune board on the body coil.

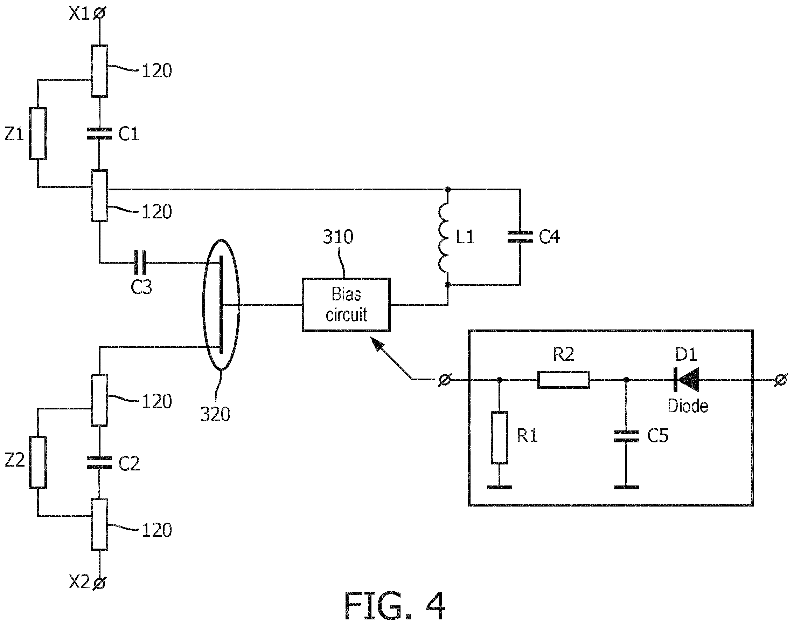

Fig. 4 illustrates an example of an electronic circuit for embodiments of the invention. The strip conductor 120 between connection points X1 and X2 is the conductor 120 of a transmit/receive coil 130. This may be a rung 340 or a rod or a ring 330 or another segment of a birdcage coil. A separate control signal 121 is injected on this conductor 120. A voltage drop via the conductor 120 or via a capacitor C is used to feed a bias network 310 for the FET 320 switch. The circuit includes chokes Z1 and Z2, inductor L1, capacitors C1, C2, C3, C4 and C5, resistors R1 and R2, as well as a diode D1.

In embodiments, FET 320 switches may optionally switch transmission lines such as coil assembly strip lines to control the current distribution on the body coil conductors 120. Such a design may reduce the electrical field and thus for safety reasons specific absorption rate in the patient by switching the transmission lines with respect to the individual patient. Additionally, this method allows to influence the resonance frequency of the individual coupled and uncoupled RF current path.

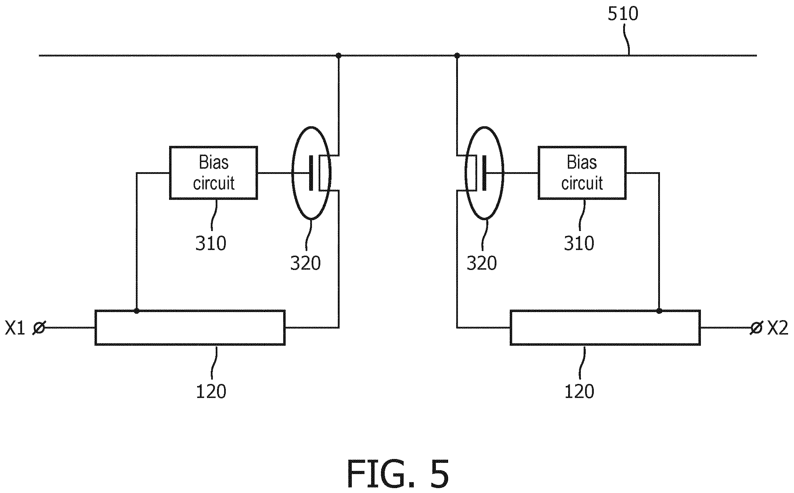

Fig. 5 illustrates a circuit diagram for such an example embodiment of the invention. In this example the FETs 320 and bias networks 310 are connected between an RF shield 510 and the RF coil conductor 120. When the FETs 320 short-circuit the RF coil 130 is active. The control signal 121 is fed via the shield 510 and RF coil strip conductor 120. To reduce distortions/coupling on the coil, the bias network 310 may be placed near the connection to the coil conductor 120. Tank circuits and/or chokes may be used to supply the DC signal to the FETs 320.

For safety reasons, it may be for various use cases and configurations be advantageous to have multiple controls of the RF coil detune status. In embodiments, the detune arrangement 110 is configured to receive a first control signal 121 via the conductor 120 as and a second control signal wirelessly or via an optical fiber 610

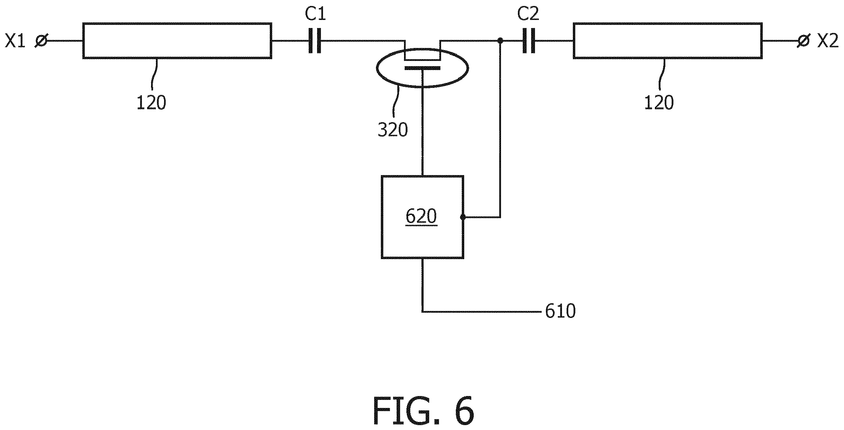

Fig. 6 illustrates a circuit diagram for an embodiment of the invention, which includes an optical fiber 610. In this example, at least one FET 320 is switched via an optical isolator 620. A signal to the optical isolator 620 is fed via an optical fiber 610. When using an optical transceiver, the FET 320 can be switched on and off. Additionally, in some embodiments, signals from local sensing, such as temperature, RF current, Gate source voltage etc., may be transmitted via a bidirectional optical fiber 610. The optical transceiver may need extra bias for power supply, which can be delivered by the RF of the active transmit/receive frequency.

A detune board may be configured such that both electrical and optical switching can be used. The FET 320 is switched via the common conductor 120 and/or simultaneously or sequentially via the optical fiber 610. If one control signal fails, there is still a second control signal, thus improving safety.

In embodiments of the invention, several FETs 320 may be connected and switched in parallel.

In embodiments, a safety monitoring arrangement on the RF coil assembly 100 monitors the balancing of the FETs 320. This allows to run the RF coil 130 safely and may enable detection or prediction that individual detuning arrangements 110 are running outside of specification, such that they can be individually serviced.

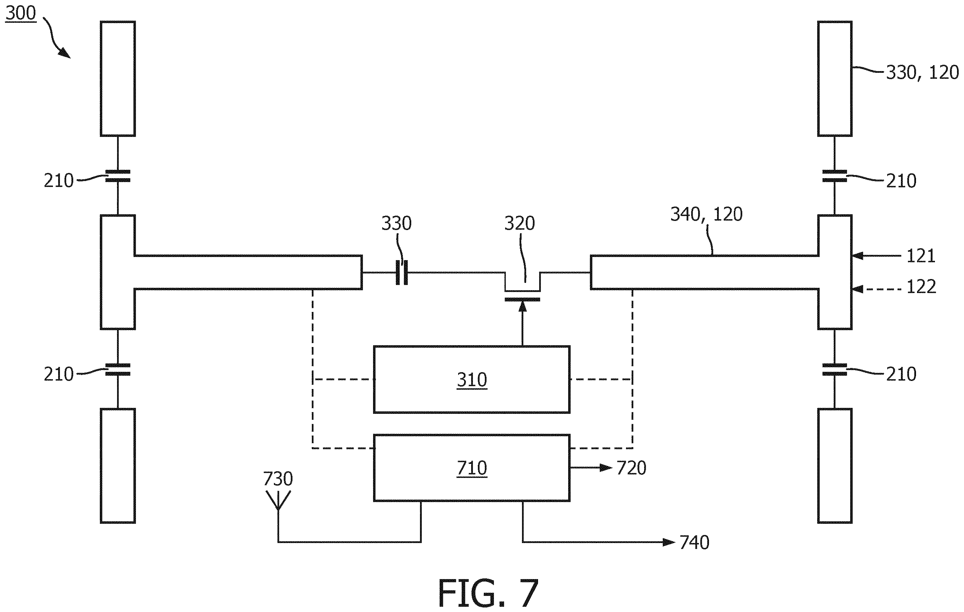

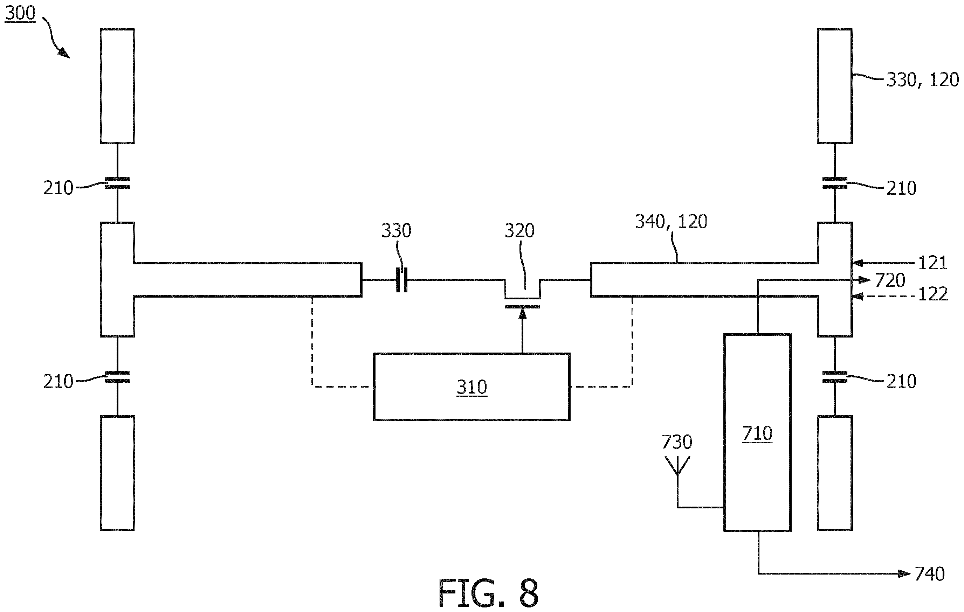

Fig. 7 and 8 show schematic illustrations of an RF transmit body coil assembly 300 with a safety monitoring arrangement according to embodiments of the invention. The safety monitoring arrangement include individual smart FET detune arrangements equipped with sensors and a microcontroller in a monitoring network 710 for detecting parameters such as detuning status, gate voltage at the gate terminal, RF current (B1 homogeneity), resonances by impedance measurement, temperature etc. A processor may control the FET detuning status and parallel FET balancing by providing adapted control signals 121 to the bias network 310 based on input from the sensors in the safety monitoring arrangement.

With reference to the example in Fig. 7, for simple function monitoring, the monitoring network 710 could be configured to modulate the current driven by the control signal 121. E.g. The control signal 121, typically a voltage in the order of some volts does drive a very low current defined by the very high gate resistance and parasitic capacitances.

The safety monitoring network 710 may be configured to enable a resistor in parallel to the gate in a repeated pattern. In such a case, the impedance of the resistor may be in the order of magnitude of the impedance of the gate resistance. This will cause a characterizing and measurable current signal at the outside of the body coil.

Such a safety monitoring signal may be transmitted for further processing from the safety monitoring network 710 via the common conductor 120. In case of any malfunction the body coil 300, the signal pattern may change or even vanish such that the signal change can be quickly detected. An encoding scheme (repetition frequency of the pattern, transient shape of the pattern, impedance of the resistor) may help to localize faults in a system with multiple monitoring networks 710, as invoked currents will linearly superimpose and a decomposition of it is straight forward when disjunct pattern are taken.

As illustrated in Fig 8, the detuning FET 320 and monitoring network 710 do not necessarily need to be at the same location of the coil. Any spot suitable to detect the RF currents of the birdcage coil by means of measuring the RF fields or electric potentials involved is suitable.

Similarly to the example above, the safety monitoring network 710 may transmit an electric monitoring signal 720 from the sensor to the processor via the common conductor 120.

As illustrated in Fig 7 and 8, the safety monitoring element may optionally also be configured to transmit a monitoring signal from the sensor wirelessly 730 or as an optical signal 740 via an optical fiber 610 to the processor.

Besides feedback control for the detuning status of the RF body coil assembly 300, the safety monitoring arrangement may also allow to process sensor data for corrective maintenance. Measuring the RF current and status of the RF body coil over time may allow to predict homogeneity as a function of time and can be used to compensate for temperature-based inhomogeneity.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

### Reference signs

- 100: RF coil assembly
- 110: Detune arrangement
- 120: Conductor
- 121: Control signal
- 122: RF signal
- 130: RF coil
- 210: Resonance capacitor
- 220: Gate capacitance
- 230: Supply voltage
- 300: Transmit body coil
- 310: Bias network
- 320: FET
- 330: Coil ring
- 340: Coil rung
- 350: Detune capacitance
- 510: RF Shield
- 610: Optical fiber
- 620: Optical isolator
- 710: Monitoring network
- 720: Electric monitoring signal
- 730: Wireless monitoring signal
- 740: Optical monitoring signal
- C: Capacitor
- R: Resistor
- L: Inductor
- D: Diode
- Z: Choke
- X: Connection points

## Claims

1. A radio frequency (RF) coil assembly (100) for use in magnetic resonance imaging, the RF coil assembly (100) comprising:
an RF coil (130) configured to receive and/or transmit an RF signal (122) input and/or output,
a detune arrangement (110) configured to receive a control signal (121) and to tune/detune the RF coil (130) to a resonance frequency based on the control signal (121), and
a conductor (120) configured to conduct the RF signal (122) and the control signal (121),
wherein the detune arrangement (110) is electrically coupled to the RF coil (130), and wherein the conductor (130) is electrically coupled to the RF coil (130) and to the detune arrangement (110), and
wherein the control signal (121) input is transmitted via the same conductor (120) as the RF signal (122) input/output.

2. The RF coil assembly (100) of Claim 1, wherein the detune arrangement (110) comprises a Field Effect Transistor (FET) (320) and a bias network (310), and wherein the bias network (310) is configured to switch the FET (320) between an open and a closed state in response to the control signal (121).

3. The RF coil assembly (100) of Claim 2, wherein the conductor (120) is galvanically isolated from the FET gate in the detune arrangement (110) and, wherein the RF coil assembly (100) is configured to divide a voltage of the control signal (121) over resonance capacitors (210) on the RF coil (130) and a FET gate capacitance (220) in the detune arrangement (110).

4. The RF coil assembly (100) according to Claim 2 or 3, wherein the detune arrangement (110) is configured such that multiple FETs (320) are switched in parallel in response to the control signal (121).

5. The RF coil assembly (100) according to any of the preceding claims, wherein the control signal (121) over the conductor (120) is a time-shifted pulsed Direct Current signal.

6. The RF coil assembly (100) according to any of the preceding claims, wherein the RF coil assembly (100) is an RF body coil assembly or an RF birdcage or a transverse electromagnetic resonator assembly coil assembly, and wherein the conductor (120) is configured to transmit RF power.

7. The RF coil assembly (100) according to any of the preceding claims, wherein the detune arrangement (110) is embedded in a printed circuit board of the RF coil assembly (100).

8. The RF coil assembly (100) according to any of the preceding claims, wherein the RF coil assembly (100) also comprises a safety monitoring arrangement configured to monitor a tuning status of the RF coil assembly (100).

9. The RF coil assembly (100) of Claim 8, wherein the safety monitoring arrangement comprises a sensor and is configured to transmit a monitoring signal from the sensor via the conductor (120).

10. The RF coil assembly (100) in Claim 8, wherein the safety monitoring arrangement comprises a sensor and is configured to transmit a monitoring signal from the sensor wirelessly or via an optical fiber (610).

11. The RF coil assembly (100) according to any of the preceding claims, wherein the detune arrangement (110) is configured to receive a first control signal (121) via the conductor (120) and a second control signal wirelessly or via an optical fiber (610).

12. A Magnetic Resonance system comprising the RF coil assembly (100) as claimed in any of the preceding claims.
